# EUROPEAN PATENT APPLICATION

(11) **EP 2 277 988 A1**
(43) Date of publication of application: **26.01.2011**
(21) Application number: 09732676.3
(22) Date of filing: 10.03.2009
(51) Int. Cl.: C12N 1/16, C12P 19/14, C12R 1/865

(54) **YEAST FOR PRODUCTION OF GLUCOSE, AND METHOD FOR PRODUCTION OF GLUCOSE USING THE SAME**

(30) Priority: 14.04.2008 JP 2008105050
(71) Applicant: Mitsui Chemicals, Inc., Tokyo 105-7117 (JP)
(72) Inventor: TAKAHASHI, Katsuyuki, Mobara-shi Chiba 297-0017 (JP); WADA, Mitsufumi, Mobara-shi Chiba 297-0017 (JP); KIMURA, Sakurako, Mobara-shi Chiba 297-0017 (JP); FUJII, Ryota, Mobara-shi Chiba 297-0017 (JP)
(74) Representative: Wills, Andrew Jonathan
(86) International application number: PCT/JP2009/054567
(87) International publication number: WO 2009/128305

(57) **Abstract**

The invention provides: yeast having cellulose degradation ability that degrades cellulose outside the cell and glucose accumulation ability that accumulates, in a reaction liquid, glucose produced from cellulose as a result of the cellulose degradation ability, when the yeast was brought into contact with cellulose in the reaction liquid; and a method for producing glucose, wherein the glucose is produced from cellulose, the method comprising the step of bringing the yeast into contact with cellulose in a reaction liquid and the step of separating and collecting, from the reaction liquid, glucose accumulated in the reaction liquid as a result of the contact of the yeast with the cellulose.

SEQUENCE LISTING

<110>  Mitsui Chemicals, Inc.

<120>  Yeast for Production of Glucose, and Method for Production of
       Glucose Using the Same

<130>  AJW/FP6732994

<140>  EP 09732676.3
<141>  2009-03-10

<150>  PCT/JP2009/054567
<151>  2009-03-10

<150>  JP 2008-105050
<151>  2008-04-14

<160>  4

<170>  PatentIn version 3.1


<210>  1
<211>  29
<212>  DNA
<213>  primer

<400>  1
cgcggcagaa acgagcaaag aaaagtaag                                          29


<210>  2
<211>  30
<212>  DNA
<213>  primer

<400>  2
agatctccat ggctcgagcg ccaaaagctc                                         30


<210>  3
<211>  50
<212>  DNA
<213>  primer

<400>  3
tacttttctt tgctcgtttc tgccgcggga gcgaccacgc tcaaggaggc                   50


<210>  4
<211>  49
<212>  DNA
<213>  primer

<400>  4
gcttttggcg ctcgagccat ggagatctag gccgaccgtg cagggcgtg                    49

## Description

### TECHNICAL FIELD

The present invention relates to yeast for producing glucose, and a glucose production method using the same.

### BACKGROUND ART

Use of biomass resources as alternatives to fossil resources has attracted attention from the viewpoints of both of efficient use of resources and environmental protection. In particular, methods in which non-edible biomass (biomass for industries), such as wood containing cellulose as a major component, is used have heretofore been intensively developed in consideration of the balance between production for foods and production for industries. In the techniques using biomass for industries, a number of technological developments have been carried out with respect to a step of isolating or purifying cellulose from biomass for industries and a saccharification step of degrading the obtained cellulose so as to convert the cellulose to monosaccharides.
Saccharification can be roughly classified into acid saccharification and enzymatic saccharification. In particular, enzymatic saccharification is preferable to acid saccharification in terms of energy consumption and reduction of environmental loads since enzymatic saccharification can be carried out at normal temperature and normal pressure, and is free of side reactions or use of dangerous chemicals.

In enzymatic saccharification, saccharification is carried out using an enzyme (cellulase) that degrades cellulose. In regard to the production of glucose from a cellulose raw material using purified cellulase, methods using an enzyme secreted by a microorganism such as a filamentous fungus belonging to *Trichoderma* (Non-patent Document 1) or a filamentous fungus belonging to *Aspergillus* (Non-patent Document 2) are known. However, processes using a purified enzyme are industrially disadvantageous in terms of the cost since the enzyme is hard to separate from the resultant glucose liquid, and thus the enzyme used has to be disposed of. Therefore, when chemical products for general purposes are desired to be produced using glucose as a raw material, an innovative process development is indispensable.
It is possible to make a microorganism produce a large amount of cellulase by using a gene recombination technique. However, production of cellulase inside a cell of a microorganism has a disadvantage in that the cellulose raw material, which is the substrate, cannot contact cellulase, and therefore saccharification cannot be achieved.

As a technology overcoming these drawbacks, a yeast presenting an enzyme on a cell surface thereof, which is called arming yeast, has been developed recently (Non-patent Document 3). This employs yeast as a carrier of an enzyme, and the enzyme can be produced by a normal method for culturing yeast. Further, since the enzyme is presented on the cell surface, the substrate present in the reaction liquid can contact the enzyme. Further, since the arming yeast has aggregation properties and sedimentation properties, the arming yeast can be easily collected from the reaction liquid. Therefore, by collecting the yeast, it is possible to easily separate the enzyme from the resultant glucose liquid and to recycle and use the enzyme.

A technique of producing an optically active compound by presenting lipase on the surface (Patent Document 1), and a yeast that produces ethanol directly from starch by presenting glucoamylase that degrades starch (Non-patent Document 4) have so far been developed. Further, an arming yeast that directly produces ethanol, through fermentation, from a cellulose raw material by presenting cellulase on the cell surface has been reported (Non-patent Document 5 and Patent Document 2). However, in Non-patent Document 5, it is reported that glucose is not detected, and that the glucose produced is immediately used for ethanol production. As described above, although there are microorganisms that produce glucose from a cellulose raw material, the glucose produced is quickly metabolized by the microorganisms since glucose is an important carbon source for the life activity of the microorganisms, and it has been difficult to accumulate glucose in the reaction liquid.

Non-patent Document 1: Montenecourt, Trends in Biotechnology, Vol.1, No.5, pp.156-161 (1983)
Non-patent Document 2: Takada et al., Bioscience, Biotechnology and Biochemistry, 62 (8), pp.1615-1618 (1998)
Non-patent Document 3: Ueda et al., Journal of Bioscience and Bioengineering, Vol.90, No.2, pp.125-136 (2000)
Non-patent Document 4: Teik Seong Khaw et al., Journal of Bioscience and Bioengineering, Vol.103, No.1, pp.95-97 (2007)
Non-patent Document 5: Fujita et al., Applied and Environmental Microbiology, Vol.70, No.2, pp. 1207-1212 (2004)
Patent Document 1: Japanese Patent Application Laid-Open (JP-A) No. 11-290078

### DISCLOSURE OF THE INVENTION

### PROBLEM TO BE SOLVED BY THE INVENTION

As described above, industrial application of methods using a purified enzyme is generally disadvantageous in terms of the cost. Further, an arming yeast presenting cellulase on a cell surface layer thereof immediately utilizes the generated glucose due to the sugar metabolism function of the yeast, as a result of which glucose cannot be accumulated. Therefore, development of a biocatalyst that shows cellulose degradation activity and that does not utilize the generated glucose has been strongly desired. In addition, development of an enzyme that can be used repeatedly has also been strongly desired.
The present invention aims at providing a yeast for producing glucose that is capable of efficiently producing glucose from cellulose at high yields, and that can be used repeatedly, and a glucose production method using the same.

### MEANS FOR SOLVING THE PROBLEM

The present invention has been made in view of the above circumstances, and provides a yeast for producing glucose, and a glucose production method using the yeast.
A first aspect of the invention provides a yeast having a cellulose degradation ability that degrades cellulose outside a cell thereof and a glucose accumulation ability that accumulates, in a reaction liquid, glucose produced from cellulose as a result of the cellulose degradation ability when the yeast contacts with cellulose in the reaction liquid.
A second aspect of the invention provides a glucose production method of producing glucose from cellulose, the method comprising the step of contacting the yeast with cellulose in a reaction liquid, and the step of separating and collecting, from the reaction liquid, glucose produced and accumulated in the reaction liquid as a result of the contact between the yeast and the cellulose.

### EFFECT OF THE INVENTION

According to the invention, yeast for producing glucose that is capable of efficiently producing glucose from cellulose at high yields and that can be used repeatedly, and a glucose production method using the same are provided.

### BRIEF DESCRIPTION OF THE DRAWINGS

Fig. 1 is a graph showing changes in the amount of glucose consumed by the arming yeast at the respective temperatures employed in Examples of the invention.
Fig. 2 is a graph showing changes in the amount of glucose accumulated by the arming yeast at the respective temperatures employed in Examples of the invention.

### BEST MODE FOR CARRYING OUT THE INVENTION

### <Yeast for Producing Glucose>

The yeast according to the invention is a yeast having a cellulose degradation ability that degrades cellulose outside a cell thereof and a glucose accumulation ability that accumulates, in a reaction liquid, glucose produced from cellulose as a result of the cellulose degradation ability when the yeast contacts with cellulose in the reaction liquid.

Since the yeast according to the invention (hereinafter referred to as yeast for producing glucose) has an ability that degrades cellulose outside the cell and glucose accumulation ability that accumulates glucose in a reaction liquid when the yeast contacts with cellulose in the reaction liquid, the yeast according to the invention is capable of producing glucose outside the cell in the reaction liquid and accumulating the resultant glucose in the reaction liquid.
Further, since the yeast for producing glucose itself has an ability that degrades cellulose outside the cell, reuse of the enzyme is facilitated by recovering and reusing the yeast rather than the enzyme as a compound.
From these reasons, glucose which is advantageous in terms of the cost can be produced at high yields.

The scope of the term "step" as used in the invention is not restricted to an independent step, and also includes cases in which the step is not clearly distinguishable from other steps as long as the predetermined action of the step is achieved.
When a numerical range is described in the present specification, the range includes the value described at the left (the minimum value) and the value described at the right (the maximum value), unless otherwise specified.

In the invention, cellulose means a chain macromolecular substance in which D-glucose units are linked via β-1,4-glucoside bonds, and has both a reducing terminal and a non-reducing terminal. Cellulose molecules are highly aligned in cellulose fibers, thereby forming crystalline regions; however, the alignment is disordered at various portions, and thus non-crystalline regions and/or regions similar thereto are formed.
Natural celluloses are known to have a crystallinity of about 70%, and regenerated celluloses are known to have a crystallinity of about 40%, according to an X-ray method.

In regard to the crystal structure of cellulose, each natural cellulose shows a structure called "cellulose I", wherein the cellulose forms not only chains but also lateral bondings to form a bundle, thereby forming a microfibril. Currently, celluloses are considered to have four crystalline forms; thus, in general, cellulose is roughly classified into I, II, III and IV. Regenerated celluloses are obtained by strongly swelling and dissolving cellulose I using a high-concentration salt solution, or an acid or an alkali, and forming cellulose again therefrom. Regenerated celluloses have the crystal form of cellulose II. Celluloses obtained by immersing cellulose I or II in liquid ammonia and gradually regenerating cellulose, or by degrading an amine adduct compound, has the form of cellulose III. Further, cellulose IV is a crystal structure obtained by heating cellulose II to a high temperature of 250°C or higher in glycerin. The scope of cellulose in the invention encompasses those showing any of these cellulose patterns.

Further, the scope of cellulose in the invention encompasses cellulose derivatives. Examples of cellulose derivatives that may be selected include cellulose esters such as cellulose acetate, cellulose propionate, cellulose butyrate, cellulose acetate butyrate, cellulose nitrate, cellulose sulfate and cellulose phosphate; cellulose ethers such as methylcellulose, ethylcellulose, benzylcellulose, carboxymethylcellulose, hydroxymethylcellulose and carboxyethylcellulose; and viscose.

Cellulose degradation in the invention means a reaction whereby a glucoside bond of cellulose is cleaved; the glucoside bond to be cleaved may be located at a crystalline region or a non-crystalline region, and may be located at either a non-reducing terminal or a reducing terminal of the cellulose fiber or at a region therebetween. The size of the cellulose crystal is not restricted as long as cellulase or another catalyst can be adsorbed on the surface of the cellulose and can exert a molecular-chain cleaving function.

The yeast of according to the invention having a cellulose degradation ability that degrades cellulose outside the cell may be any yeast having a cellulolytic enzyme activity outside the cell, and examples of such a yeast include an arming yeast presenting a cellulolytic enzyme at a surface layer thereof. By using the arming yeast, the cellulose degradation ability can be efficiently imparted to the surface of the yeast, and handling of the enzyme may be facilitated.

### (Arming Yeast)

As used in the invention, arming yeast refers to a yeast cell presenting, at a surface layer of the cell, a functional protein or peptide fused to a protein localized at the cell surface layer, thereby acquiring functionality derived from the protein or peptide presented.
With respect to methods for preparing an arming yeast, several methods are currently known, which may be selected in accordance with the type of the protein localized at the cell surface layer of the yeast.

Examples thereof include a method using α-agglutinin as the protein localized at the cell surface layer. This is a technique whereby a gene encoding α-agglutinin of which the C-terminal region is added with a GPI (Glycosyl Phosphatidyl Inositol)-anchor adhesion signal is fused to a gene encoding a functional protein of interest, and is allowed to be expressed, thereby presenting the functional protein at the cell surface layer. Another method uses FLO protein as the protein localized at the cell surface layer. FLO protein is an aggregating protein of yeast and bound to the cell wall via hydrophobic bonding. Fusion of the gene encoding the FLO protein (*Flo1*) to a gene encoding a functional protein, and expression thereof presents the functional protein at the surface layer. In still another method, Pir (Protein Internal Repeat) is used as the protein localized at the cell surface layer. Pir is a protein covalently bound to the cell wall of yeast. Similarly to the above method, fusion of Pir to a functional protein and expression thereof presents the functional protein at the cell surface layer.
The arming yeast in the invention may be prepared by any of the above methods.

The arming yeast in the invention is preferably an arming yeast produced by using α-agglutinin, since such an arming yeast is capable of stably presenting a high-molecular-weight protein at the surface layer. For example, it is preferable to use the arming yeast of the MT8-1/pBG211/pEG23u31H6/pFCBH2w3 strain described in Appl. Environ. Microbiol., Vol.70(2), pp.1207-1212 (2004).
The yeast that serves as the carrier of the enzyme is not restricted as long as it allows arming, and examples thereof include those belonging to *Saccharomyces* and *Pichia.* Among yeasts, yeasts of *Saccharomyces,* which have made successes, are expected to exhibit stable enzymatic activity.

The arming yeast in the invention preferably has cellulase, which degrades cellulose, as the functional protein to be presented at the cell surface layer.
In general, cellulase refers to β-1,4-glucanase. In the invention, cellulase means a group of enzymes that cleave a β-1,4-glucoside bond to generate glucose from cellulose, and examples thereof include endo-β-1,4-glucanase (which is also referred to as carboxymethylcellulase), exo-β-1,4-glucanase (which is also referred to as cellobiohydrolase) and β-glucosidase.

The sources of these enzymes are not particularly restricted, and examples of the sources include microorganisms of the genera of *Trichoderma, Aspergillus, Thermotoga, Candida, Saccharomyces, Humicola, Irpex, Talaromyces, Phanerochaete, Piromyces, Orpinomyces, Neocallimastix, Streptomyces, Penicillium, Cryptococcus, Cellulomonas, Clostridium, Thermobifida, Bacillus, Thermoascus, Fusicoccum, Ruminococcus* and *Vibrio,* and insects. When enzymes are used in combination, the sources of the enzymes may be mutually the same or different.

In the invention, the cellulase may be used singly or in combination of two or more thereof. In particular, a combination of endoglucanase, cellobiohydrolase and β-glucosidase is preferred from the viewpoint that cellulose or intermediate degradation compounds of cellulose can be efficiently degraded to produce glucose.
When plural cellulases are used, presenting at least one type of cellulase at the cell surface layer of the yeast is sufficient, and a free cellulase may be used additionally therewith. From the viewpoint of the recovery efficiency and the reuse efficiency of the enzyme, it is preferable to use a yeast presenting the cellulase to be used at the cell surface layer. When a free cellulase is used, concentrations at which the cellulase is usually used are directly applicable.

The yeast according to the invention has, in addition to the cellulose degradation ability described above, a glucose accumulation ability that accumulates glucose in the reaction liquid. Here, "accumulate glucose in the reaction liquid" means that the cellulose-derived glucose, which has been efficiently generated outside the cell, is not effectively utilized. That is, even though the glucose is present outside the yeast cell in a state in which the glucose can contact the yeast, the glucose remains rather than being utilized by the yeast, and an amount of glucose can be confirmed in the reaction liquid. Here, the glucose amount may be, for example, such that, after contacting an arming yeast at a concentration of 3% (w/v) with 5 g/L glucose in a reaction liquid for 60 hours or longer, the amount of glucose present in the reaction liquid is 0.3 g/L or more.

The yeast by which the glucose is not effectively utilized is preferably a yeast of which the glucose metabolic activity is decreased. Use of a yeast of which the glucose metabolic activity is decreased reduces consumption of the generated glucose by the yeast, thereby enabling effective accumulation of glucose in the reaction liquid.

Here, "the glucose metabolic activity is decreased" means that the glucose uptake speed of the yeast of interest is smaller than the maximum glucose uptake speed of the yeast. The decrease in the glucose uptake speed of the yeast may be a temporary decrease or a semipermanent decrease. Here, the maximum glucose uptake speed is the speed observed in a case in which the yeast is cultured in a medium containing sufficient nutrient sources in an optimum temperature condition under sufficient aeration. Such a case may be, for example, a case in which the yeast is cultured in YPD medium at a culture temperature of from 30°C to 35°C under sufficient aeration, and the yeast that has grown sufficiently thereby is collected, suspended in water containing glucose, and cultured at a temperature of from 30°C to 35°C.

The scope of "decrease" of the glucose metabolic activity in the invention encompasses not only a case in which the innate glucose metabolic activity of the yeast is decreased compared to an ordinary state, but also a case in which the metabolic activity is lost and glucose uptake does not occur at all.

Examples of the yeast of which the glucose metabolic activity is decreased include a yeast in which a decrease in glucose metabolic activity has been induced by heat treatment, a yeast in which a decrease in glucose metabolic activity has been induced by treatment with a surfactant, and a yeast in which a decrease in glucose metabolic activity has been induced by exposure to an antibiotic substance or UV.

The heat treatment for inducing a yeast to have a decreased glucose metabolic activity is treatment at a temperature exceeding the optimum growth temperature of the yeast. A temperature exceeding the optimum growth temperature is preferred since the glucose metabolic activity can be surely decreased at such a temperature. Here, the "optimum growth temperature of the yeast" means a temperature at which the growth rate of the yeast of interest is highest. The temperature condition that exceeds the optimum growth temperature of the yeast varies depending on the type of yeast used, and is generally a temperature higher than 30°C, preferably a temperature higher than 35°C from the viewpoint of decreasing the glucose consumption, and more preferably 40°C or higher from the viewpoint of ensuring suppression of the glucose consumption.

From the viewpoint of maintaining the activity of cellulase, it is more preferable that the temperature of the heat treatment is higher than the optimum growth temperature of the yeast, but lower than the denaturation temperature of the cellulase. This ensures induction of a decrease in glucose metabolism, and also maintains a high enzyme activity. Here, the denaturation temperature of cellulase means a temperature at which the cellulase ceases to exhibit it activity due to heat denaturation. Since the optimum temperature varies depending on the type of cellulase, the temperature of the heat treatment may be appropriately selected in accordance with the cellulase used; for example, in the case of the cellulase of *Thermotoga* sp., the temperature is preferably not more than 105°C. From the viewpoint of ensuring suppression of activity decreases of many cellulases, the temperature is preferably not more than 90°C, more preferably not more than 80°C, most preferably not more than 70°C.

In order to allow cellulase to sufficiently exert the enzyme function thereof in a yeast in which a decrease in the glucose metabolism activity has been induced, it is preferable that the morphology of the yeast, which is the carrier, is maintained normal. Therefore, the temperature of the heat treatment is more preferably higher than the optimum growth temperature of the yeast, but lower than the heat denaturation temperature of the yeast. Here, the heat denaturation temperature of the yeast means a temperature at which the yeast becomes unable to maintain its normal morphology due to heat denaturation. The temperature of the heat treatment is preferably not more than 70°C, more preferably not more than 60°C, most preferably not more than 50°C, from the viewpoint of ensuring that the morphology of the yeast is maintained, although the temperature varies depending on the type of yeast. For example, in the case of an arming yeast in which an endoglucanase and a cellobiohydrolase which are derived from *Tricoderma reesei* and a β-glucosidase derived from *Aspergillus aculeatus* are presented at the surface layer of the *Saccharomyces cerevisiae* yeast, the temperature of the heat treatment is preferably not more than 50°C.

In consideration of the above, the yeast may have a glucose accumulation ability that has been induced heat treatment at a temperature of preferably from 40°C to 70°C, more preferably from 40°C to 60°C, and still more preferably from 40°C to 50°C, depending on the temperature range within which the morphology of the yeast can be maintained and the activity of the cellulase can be maintained. Thus, the glucose accumulation ability is most preferably induced by heat treatment at a temperature range of from 40°C to 50°C, from the viewpoints of ensuring the induction of a decrease in glucose metabolism and accumulating glucose in the reaction liquid at high yields.
Although the duration of the heat treatment for the induction varies depending on the temperature, the type of yeast and the like, the duration may be generally from 1 to 120 hours, and may be from 3 to 48 hours from the viewpoint of ensuring the induction.

The surfactant used for the treatment with a surfactant for inducing the yeast to have a decreased glucose metabolic activity may be an ionic surfactant or a nonionic surfactant. The surfactant is preferably a nonionic surfactant, which is less likely to adversely affect biomolecules.

The nonionic surfactant is preferably an ester-type or ester-ether-type nonionic surfactant from the viewpoint of the effect with respect to the induction of a decrease in glucose metabolism, and examples thereof include polyoxyethylene nonylphenyl ether, polyoxyethylene sorbitan trioleate, sorbitan sesquioleate, 4-(1,1,3,3-tetramethylbutyl)phenyl-polyethyleneglycol, and sorbitan monooleate. Such surfactants are commercially available under the trade names of, for example, NONIDET P-40, TWEEN 85, ARLACEL C, TRITON X-100, SPAN 80 and the like.

The concentration of surfactant for inducing a decrease in glucose metabolic activity is preferably from 0.015 to 10% (w/v), more preferably from 0.02 to 5% (w/v), and still more preferably 0.05 to 1% (w/v). The percentage used to represent the concentration of surfactant is % by mass relative to the total amount of the liquid to be treated that is to be subjected to the surfactant treatment. When the surfactant concentration is within the above range, it is ensured that a decrease in metabolic activity is induced, without largely decreasing the activity of the cellulase. The yeast for producing glucose can be obtained by contacting a surfactant at the above concentration with the yeast for from 1 to 120 hours, or, from the viewpoint of ensuring the induction, for from 3 to 48 hours.

In regard to the type and concentration of the antibiotic substance or the condition of UV irradiation for inducing a yeast to have a decreased glucose metabolic activity, those usually employed for decreasing the bioactivity without killing yeast can be applied as they are.

The yeast of which the glucose metabolic activity is decreased is preferably a yeast in which the induction has been achieved by heat treatment, surfactant treatment, or a combination thereof, from the viewpoint of the cost and the treatment efficiency. Either the heat treatment or the surfactant treatment may be carried out singly. Alternatively, one of the heat treatment or the surfactant treatment may be carried out first, followed by the other treatment. Alternatively, the heat treatment and the surfactant treatment may be carried out simultaneously. This enables a yeast having the cellulose degradation ability, of which the glucose metabolic activity is decreased, to be easily obtained.

### <Glucose Production Method>

The glucose production method according to the invention includes the step of contacting the yeast for producing glucose with cellulose in a reaction liquid (contact step); and the step of separating and collecting, from the reaction liquid, glucose produced and accumulated in the reaction liquid as a result of the contact between the yeast and the cellulose (separation/collection step).
This method enables glucose to be effectively produced outside the cell from cellulose, and the produced glucose accumulates in the reaction liquid, as a result of which glucose can be produced more efficiently than in cases in which yeasts not having the glucose accumulation ability are used.

The yeast for producing glucose used in the invention may be subjected to a preculture step in which the yeast is grown to a predetermined number, before being used in the present glucose production method.
The conditions for the preculture may be conditions in which the yeast for producing glucose, such as an arming yeast, grows to have a desired yeast weight. The culture medium may be a medium mainly consisting of a basal medium that is generally employed for culturing yeast, and any medium that is usually used according to the type of arming yeast may be employed.

The basal medium is not restricted as long as it contains a carbon source, a nitrogen source, an inorganic ion, and, optionally, other minor components. Examples of carbon sources that are used as appropriate include sugars such as glucose, fructose and molasses, organic acids such as fumaric acid, citric acid and succinic acid, alcohols such as methanol, ethanol and glycerol. Here, the carbon source is preferably a carbon source that is cheaper than glucose, and examples of the carbon source that can be used include plant-derived raw materials such as blackstrap molasses and cellulose raw materials.

Examples of general carbon sources include sugars such as starch, fructose, sucrose, xylose and arabinose; wood or harbaceous degradation products containing large amounts of such components; and cellulose hydrolysates. Further, glycerin and fatty acids derived from vegetable oils are also encompassed by the scope of the carbon source in the invention.
Examples of favorable plant-derived raw materials that can be used in the invention include crops such as cereals; maize; rice; wheat; soybean; sugarcane; beet; and cotton. The use form thereof as raw materials is not limited, and is, for example, an unprocessed product, juice or a ground product. Alternatively, any of the carbon sources described above may be used in a pure form.

Examples of nitrogen sources that are used as appropriate include inorganic and organic nitrogen sources such as organic ammonium salts, inorganic ammonium salts, nitrate nitrogen, ammonia gas, aqueous ammonia, and protein hydrolysates. Examples of inorganic ions that are used as appropriate include magnesium ion, phosphate ion, potassium ion, iron ion, and manganese ion. Examples of organic minor components that are used as appropriate include: vitamins, amino acids and the like, and yeast extracts containing these; peptone; polypeptone; corn steep liquor; and casein degradation products. In a case in which the preculture step is independent of the other steps, glucose may be used, and the use of glucose enables more efficient growth of the yeast. In a case in which glucose is contained in the culture solution for the preculture, the yeast is collected and recovered from the culture solution of the preculture step, and thereafter used for another step.

Further, the culture medium may also contain other additional components that are usually added to culture media for microorganisms, such as antibiotic substances, at usually-employed amounts. In order to suppress foaming during culturing, an anti-foaming agent may be added at an appropriate amount.

Examples of the medium used in the invention include a liquid medium based on an aqueous medium, and a solid medium based on a solid phase such as agar. Liquid media are preferred in consideration of application to industrial production. A usually-employed aqueous medium, such as distilled water or a buffer solution, may be used as the aqueous medium for forming the liquid medium.
The culture condition for culturing the yeast for producing glucose is not particularly restricted, and is, for example, an aerobic or anaerobic condition in which the pH is from pH 4 to 9, and preferably from pH 6 to 8, and the temperature is from 20°C to 40°C, and preferably from 25°C to 35°C. When the yeast for producing glucose is an arming yeast, the arming yeast may be maintained and grown under the same culture condition as that for normal yeast.

The production method according to the invention may include a pretreatment step for the preparation of the above-described yeast for producing glucose, before the contact step. This allows the preparation of the yeast for producing glucose and the glucose production using the yeast to be sequentially carried out.

The pretreatment step is preferably the heat treatment, surfactant treatment, or combination thereof as described above, and the conditions used for the production of the yeast for producing glucose described above may be directly applicable as the conditions for these treatments.
The pretreatment liquid used in the pretreatment step may not remove the cellulose degradation ability, and examples thereof include water (sterile water) and various buffer solutions such as a phosphate buffer solution (PBS), a Tris-hydrochloric acid (Tris-HCl) buffer solution, a citrate buffer solution and a phosphate-citrate buffer solution.

The temperature and pH of the pretreatment liquid may be appropriately selected in accordance with the activity of the cellulase to be used. Thus, for example, a 50 to 200 mM phosphate buffer solution (pH 4 to 7) is more preferred from the viewpoint of maintaining the activity of the cellulase of *Trichoderma.* The pretreatment liquid may alternatively be a culture solution used for normal culture as long as the decrease in the glucose metabolic activity of the yeast is not impaired.
By applying such a pretreatment step, the glucose metabolic activity can be decreased.

The duration of the pretreatment varies depending on the type of cellulase presented at the surface layer of the arming yeast, and thus cannot be specified generally. In general, longer pretreatment time leads to a larger decrease in the glucose utilization capability of the arming yeast. However, the pretreatment time is preferably limited from the viewpoint of maintaining the enzyme activity of the cellulase. From this viewpoint, for example, the pretreatment time is preferably from 3 to 24 hours in the case of *Trichoderma-*derived cellulase.

In a case in which the arming yeast after the preculture step is subjected to the pretreatment step, it is preferable to collect the cells from the culture and suspend the cells in a reaction liquid as described above such as water or an appropriate buffer solution, and then use the cells in the pretreatment step, in order to suppress degradation of the cellulase presented on the cell surface. This ensures that the degradation of the cellulase by proteolytic enzymes, such as proteases, secreted by the yeast is suppressed

### (Contact step)

In the contact step in the invention, the yeast according to the invention described above is contacted with cellulose in a reaction liquid. Here, the contact may be carried out by adding the yeast into a reaction liquid containing cellulose, or by adding cellulose into a reaction liquid containing the yeast.

Examples of the cellulose used in the invention as a raw material for producing glucose include, in addition to pure cellulose, a wide range of biomass materials such as wood, bagasse and corn, which contain cellulose as a component, and also pulp obtained from wood or the like.
Pulp is a general term for materials extracted as fibrous materials from wood and the like, and containing cellulose as the main component. Pulps are roughly classified into chemical pulps produced by removing lignin by using chemicals; mechanical pulps produced by pulping using a mechanical force of a grinder or a refiner without removing lignin; chemimechanical pulps produced by using both of the methods; and recycled pulps. The cellulose that can be used as a raw material in the invention may be any of these types of pulp, or a combination of two or more thereof.

The raw material of the pulp may be a wood material or a non-wood material. Examples of the wood material include conifers and broad-leaved trees, and examples of the non-wood material include rice straw, wheat straw, bagasse, bamboo, various hemps, cotton linter, kenaf, paper mulberry and *Edgeworthia chrysantha.* The raw material for the pulp may be a combination of these. Further, the pulp may be a bleached pulp produced by bleaching, or an unbleached pulp.
Therefore, the cellulose used in the invention as a raw material for producing glucose may contain, in addition to cellulose, hemicellulose, lignin or the like. Cellulose materials from which a sugar that can be used for fermentation, such as glucose or xylose, can be finally obtained by hydrolysis are encompassed by the scope of the cellulose in the invention, without particular restrictions.

The reaction liquid for cellulose degradation is not restricted as long as the yeast according to the invention can exhibit cellulose degradation ability therein, and may be water or a buffer solution. Examples thereof include those described above in the description of the pretreatment step.

### (Reaction Step)

In the reaction step in the invention, glucose is produced from cellulose by the yeast according to the invention having the cellulose degradation ability that degrades cellulose outside the cell, and having the glucose accumulation ability that accumulates, in a reaction liquid, the glucose produced from cellulose as a result of the cellulose degradation ability when the yeast contacts with cellulose in the reaction liquid. The reaction time therefor cannot be specified generally since it varies depending on the type of cellulase presented at the cell surface layer and the addition amount of the arming yeast that has been subjected to the pretreatment. In general, the maximum glucose accumulation is attained at a reaction time of from 24 hours to 250 hours in many cases.
Examples of the mode of the reaction include a batch reaction in which reaction components are added and allow to undergo the reaction, and a continuous reaction in which the reaction is continuously carried out while removing the generated glucose by using a UF membrane or the like.

In regard to the reaction temperature and pH in the reaction step, it is preferable to employ a reaction temperature or pH unsuitable for the growth of the yeast according to the invention in order to prevent the glucose metabolic capability that has been decreased by the pretreatment from being recovered in a short time.
It is particularly preferable to employ a temperature or pH similar to that employed in the pretreatment step.

### (Separation and Collection Step)

When the reaction step is finished, glucose has accumulated in the reaction liquid at a high concentration. Separation and collection of the glucose from the reaction liquid may be carried out by a usually-employed method, and examples thereof include filtration with a filter, centrifugal separation and chromatography.

Even after the reaction step is finished, the yeast according to the invention can be used repeatedly for reaction steps in which glucose is produced from cellulose. That is, the production method according to the invention may further include a step of contacting the yeast in the reaction liquid after the separation and collection step with newly-supplied cellulose (reuse step). This further increases the yield of glucose by repeatedly using the yeast for producing glucose according to the invention.

In the invention, the scope of the term "use repeatedly" encompasses a case in which, in a batch reaction, yeast in the reaction liquid after the reaction step is finished is collected and then used by being contacted with a new reaction liquid containing cellulose, as well as a case in which, in a continuous reaction, the yeast contacts with newly-supplied cellulose throughout the reaction step.

In regard to the manner of counting the number of repetitions, in the case of a batch reaction, the time when a new reaction liquid containing cellulose contacts with the yeast used in the previous batch is counted as one repetition; in the case of a continuous reaction, the time when newly-supplied cellulose contacts with the yeast that was present at the initiation of the reaction step is counted as one repetition. The number of repetitions may be one or more in both of the batch reaction and the continuous reaction, and there is no upper limit..

The production method according to the invention may include collecting the yeast in the reaction liquid after the separation and collection step. The above-described methods in the description of the step of separation and collection from glucose are directly applicable as the method for collecting the yeast. This enables the yeast for producing glucose according to the invention to be stored until the time of reuse. The step of collecting the yeast may be carried out at any time after the reaction step, and may be carried out simultaneously with the step of separation and collection from glucose. With regard to the method for storing the yeast for producing glucose, conditions usually employed for storing yeast are directly applicable.

### EXAMPLES

The invention is described by way of examples below. However, the invention is not restricted by these Examples. Unless otherwise specified, "%" in the examples is based on weight (mass).

### Example 1

### (Decrease in glucose metabolic capability of saccharomyces arming yeast presenting cellulase at surface layer)

According to the method described in Appl. Environ. Microbiol., Vol.70(2), pp.1207-1212 (2004), an arming yeast in which an endoglucanase and a cellobiohydrolase which are derived from *Tricoderma reesei,* and an *Aspergillus aculeatus-*derived β-glucosidase are presented at the surface layer of *Saccharomyces cerevisiae* yeast (MT8-1/pBG211/pEG23u31H6/pFCBH2w3 strain described in the above document) was obtained.

The glucose metabolic activity of this arming yeast was decreased in the following manner.

### (Culturing Step)

Using a colony grown on SD agar medium (free of Trp, ura and His) as an inoculum, preculture was carried out in 5 mL SD medium (free of Trp, ura and His) at a culture temperature of 30°C and a rotation speed of 150 rpm for 1 day while shaking. 3 mL of the resulting culture was inoculated into 500 mL SD medium (free of Trp, ura and His) in a 2-L baffle flask, followed by culturing at a culture temperature of 30°C and a rotation speed of 150 rpm for 3 days while shaking.

### (Washing Step)

The thus cultured arming yeast was separated from the culture solution by centrifugal separation at a rotation speed of 5000 rpm (using HITACHI HIMAC CR22G) at 4°C for 10 minutes, and the collected yeast was suspended in 200 mL pure water, followed by centrifuging under the above centrifugal separation conditions, thereby washing the yeast. The washing was carried out once again, that is, twice in total. Thereafter, the collected arming yeast was resuspended in 300 mL pure water.

### (Pretreatment Step)

The thus prepared suspension of the arming yeast was aliquoted in 30 mL volumes into 50-mL flasks, and incubated at temperatures of 30°C, 35°C, 40°C and 45°C for 5 hours at a rotation speed of 120 rpm.

### (Reaction Step)

Thereafter, glucose (manufactured by Wako Pure Chemical Industries, Ltd.) was added thereto so as to have a concentration of 2% by weight, followed by incubation for 24 hours at respectively the same temperatures (30, 35, 40 and 45°C) as in the pretreatment step.

### (Measurement of Glucose Amount)

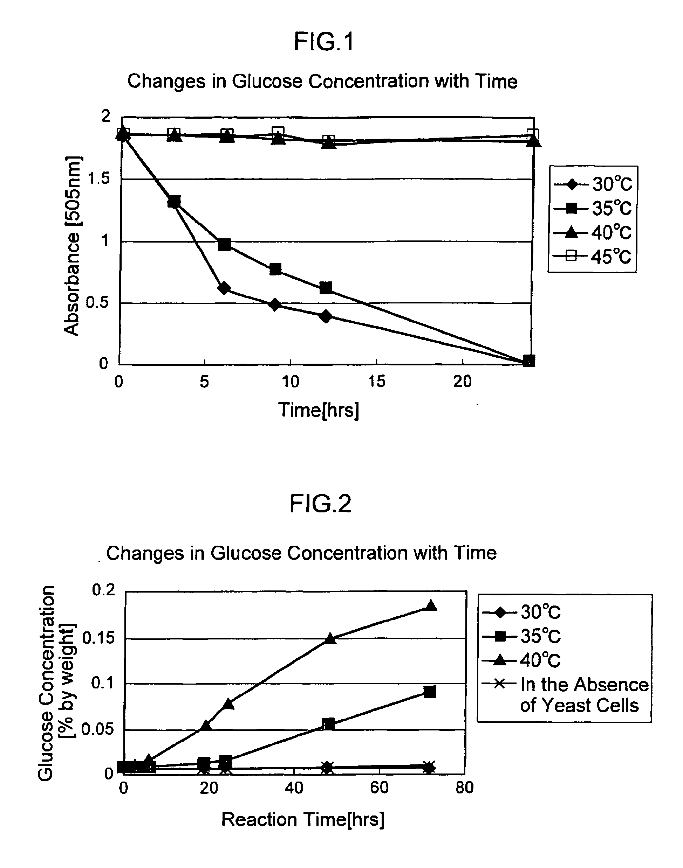

The amount of glucose present in the reaction liquid was monitored over time. The concentration of glucose was determined by using a Glucose CII-Test Wako kit, which is commercially available from Wako Pure Chemical Industries, Ltd., and measuring the absorbance at 505 nm. The results are shown in Table 1 and Fig. 1. The values in Table 1 are absorbances at OD 505 nm.

As shown in Table 1, at 30°C, which is the same temperature as the culture temperature, a decrease in the glucose concentration with time was observed, and most of the 2% by weight glucose was consumed by 24 hours. In the case of the incubation at 35°C, although the decrease rate was lowered compared to that at 30°C, a decrease in the glucose concentration with time was observed, and most of the glucose was consumed by 24 hours, similarly to the case of 30°C. In contrast, it was observed that the arming yeasts incubated at 40°C and 45°C hardly consumed glucose by 24 hours.

**Table 1**

| Pretreatment liquid | 300 ml | | | | | |
|---|---|---|---|---|---|---|
| Incubation time after glucose addition [hrs] | 0 | 3 | 6 | 9 | 12 | 24 |
| 30°C | 1.89 | 1.32 | 0.63 | 0.50 | 0.41 | 0.04 |
| 35°C | 1.87 | 1.33 | 0.98 | 0.78 | 0.63 | 0.04 |
| 40°C | 1.87 | 1.86 | 1.86 | 1.85 | 1.82 | 1.85 |
| 45°C | 1.86 | 1.86 | 1.86 | 1.88 | 1.82 | 1.88 |

### Example 2

### (Production of glucose from cellulose raw material by saccharomyces arming yeast having decreased glucose metabolic capability)

In the same manner as in the above Example 1, the arming yeast presenting cellulase at the cell surface layer (MT8-1/pB0211/pEG23u31H6/pFCBH2w3 strain) was cultured, washed, and subjected to the pretreatment step. However, the pretreatment was carried out at pretreatment temperatures of 30°C, 40°C and 45°C for a treatment time of 3 hours. A reaction step was carried out in the following manner.

### (Reaction Step)

A cellulose swollen by phosphoric acid was added so as to have a concentration of 0.6% by weight. Subsequently, the resultant was incubated at temperatures of 30°C, 40°C and 45°C for 24 hours. A control group was prepared by adding the cellulose swollen by phosphoric acid to 50 mL of water that did not contain the yeast cells, and treating the resultant at 45°C in the same manner as above.

Here, the cellulose swollen by phosphoric acid was prepared as follows. 4g of cellulose (AVICEL PH-101, manufactured by Fluka) was added to 100 mL of ice-cooled H₃PO₄ (manufactured by Nacalai Tesque, 85% purity), and the resulting mixture was stirred overnight to achieve complete dissolution. Thereafter, the cellulose liquid after dissolution was slowly poured into 1900 mL of ice-cooled water while stirring, followed by stirring for 1 hour. The cellulose liquid was subjected to suction filtration with a Buchner funnel (filter paper: quantitative filter paper No. 6; manufactured by ADVANTEC), and the cellulose was washed and neutralized in the following manner. First, the cellulose was subjected to washing treatment by passing 50 mL of pure water four times, and then subjected to neutralization treatment by passing 50 mL of 1%NaHCO₃ twice. Finally, 50 mL of pure water was passed through three times. The pellet was then suspended in 50 mL of pure water, and treated using a homogenizer (SONIFIER CELL DISRUPTOR 200 manufactured by BRANSON) for a treatment time of 2 minutes three times, as a result of which a cellulose. swollen by phosphoric acid was obtained. A part of the cellulose swollen by phosphoric acid was taken and dried in a drying chamber, dry weight was measured, and the cellulose concentration was calculated.

### (Measurement of Glucose Amount)

The amount of glucose present in the reaction liquid was monitored over time. The glucose amount was determined by using a Glucose CII-Test Wako kit, which is commercially available from Wako Pure Chemical Industries, Ltd., and measuring the absorbance at 505 nm. The results are shown in Table 2 and Fig. 2. As shown in Table 2 and Fig. 2, glucose did not accumulate in the case in which the pretreatment and the reaction were conducted at 30°C; however, glucose accumulated in the case in which the pretreatment and the reaction were conducted at 40°C and the case in which the pretreatment and the reaction were conducted at 45°C, and the glucose concentrations after 72 hours were 0.093% by weight and 0.187% by weight, respectively.

**Table 2**

| Reaction time | Amount of glucose generated [% by weight] | | | |
|---|---|---|---|---|
| [hrs] | 30°C | 40°C | 45°C | Without yeast cells |
| 0 | 0.01 | 0.01 | 0.01 | 0.01 |
| 3 | 0.01 | 0.01 | 0.01 | 0.01 |
| 6 | 0.01 | 0.01 | 0.02 | 0.01 |
| 19 | 0.01 | 0.01 | 0.06 | 0.01 |
| 24 | 0.01 | 0.02 | 0.08 | 0.01 |
| 48 | 0.01 | 0.06 | 0.15 | 0.01 |
| 72 | 0.01 | 0.09 | 0.19 | 0.01 |

### Example 3

### (Production of glucose from cellulose raw material by Saccharomyces arming yeast having a decreased glucose metabolic capability)

In the same manner as in the above Example 1, the arming yeast presenting cellulase at the cell surface layer (MT8-1/pBG211/pEG23u31H6/pFCBH2w3 strain) was cultured, washed, and subjected to the pretreatment step. However, the pretreatment was carried out at a pretreatment temperature of 45°C for a treatment time of 5 hours.
The reaction step was carried out in the same manner as in Example 2. However, the cellulose swollen by phosphoric acid was added to have a concentration of 1% by weight, the reaction temperature was 45°C, and the reaction time was 72 hours. The glucose amount measurement was conducted using a Glucose CII-Test Wako kit, which is commercially available from Wako Pure Chemical Industries, Ltd., and the glucose amount was calculated by measuring the absorbance at 505 nm. The results are shown in Table 3.

As shown in Table 3, 0.45% by weight glucose was produced in 24 hours. As the reaction time extends, 0.55% by weight glucose accumulated in 48 hours, and 0.6% by weight glucose, which corresponds to 60% of the amount of cellulose added, accumulated in 72 hours.

**Table 3**

| Reaction time [hrs] | Glucose concentration [% by weight] |
|---|---|
| 0 | 0.00 |
| 5 | 0.15 |
| 24 | 0.45 |
| 48 | 0.55 |
| 72 | 0.60 |

### Example 4

### (Decrease in glucose metabolic capability of arming yeast presenting cellulase at surface layer caused by surfactant treatment)

An arming yeast presenting, at the surface layer thereof, cellulase derived from a cellulase gene introduced into its genome was prepared, and the effect of surfactant treatment with respect to induction of a decreased in glucose metabolic capability was examined as follows. <1> Construction of arming Yeast (MT8-1/EG, CBH, BGL strain) presenting, at the surface layer thereof, an endoglucanase and a cellobiohydrolase which are derived from *Tricoderma reesei* and a β-glucosidase derived from *Aspergillus aculeatus* by introduction of the genes encoding the endoglucanase and the cellobiohydrolase derived from *Tricoderma reesei* and the β-glucosidase derived from *Aspergillus aculeatus* into the genome of *Saccharomyces cerevisiae* yeast.

In each of pBG211 (having a fusion gene cassette composed of a secretion signal, the β-glucosidase gene and a 3'-half of α-agglutinin gene), pEG23u31 H6 (having a fusion gene cassette composed of a secretion signal, a His tag, the endoglucanase II gene and a 3'-half of α-agglutinin gene) and pFCBH2w3 (having a fusion gene cassette composed of a FLAG peptide tag, the CBH II gene and a 3'-half of the α-agglutinin gene), which are plasmids described in Appl. Environ. Microbiol., Vol.70(2), pp. 1207-1212 (2004), the promoter and terminator regions of the gene cassette were replaced with the PGK promoter and the PGK terminator, respectively, according to a method similar to that described in a document by Suihko et al. (Suihko et al., Applied and Environmental Microbiology, Vol.35, pp.781-787 (1991)) using restriction sites. The obtained plasmids were named pBG211-2, pEG23u31H6-2 and pFCBH2w3-2, respectively.

A series of regions composed of the promoter, the gene cassette containing the endoglucanase II gene, and the terminator in pEG23u31H6-2 was amplified by PCR under normal conditions. Both ends of the resultant DNA fragment and the downstream of the terminator region of pBG211-2 were cleaved with a restriction enzyme, and linked with DNA ligase. *E. coli* DH5α competent cells were transformed using the DNA obtained, the resulting transformants were applied to an LB plate containing ampicillin, and colonies were obtained. The plasmid was recovered from this *E. coli* according to a common method, as a result of which a plasmid having the gene cassette containing the endoglucanase and the gene cassette containing the β-glucosidase was obtained.

This plasmid was cleaved at a restriction site in the His3 marker, and used to transform a *Saccharomyces cerevisiae* MT8-1 strain prepared using a Fast Yeast Transformation Kit (manufactured by Takara Bio Inc.). The transformants were applied to a SD plate medium that did not contain histidine and were cultured at 30°C for 3 days, thereby preparing a yeast of which the genome had the endoglucanase gene and the β-glucosidase gene incorporated thereto.

Competent cells were prepared from the obtained yeast, using a Fast Yeast Transformation Kit. The competent cells were then transformed with a DNA fragment prepared by cleaving pFCBH2w3-2 at a restriction site in the Trp1 marker. The transformants were applied to a SD plate medium that contained neither tryptophan nor histidine, and were cultured at 30°C for 3 days, as a result of which a yeast to which three types of enzyme―the endoglucanase, the β-glucosidase and the cellobiohydrolase - were incorporated was obtained. This yeast was named "MT8-1/EG, CBH, BGL strain".

### <2> Induction of decrease in glucose metabolic function in arming yeast by surfactant treatment

In the same manner as in the above Example 1, the arming yeast presenting cellulase derived from a gene in its genome at the cell surface layer (MT8-1/EG, CBH, BGL strain) was cultured and washed. However, the culture medium used was a YPD medium. Further, the pretreatment step was carried out according to any one of the following four types of procedure:
(1) a surfactant (Triton X-100, manufactured by Wako Pure Chemical Industries, Ltd.) was added to the pretreatment liquid at a concentration of 0.1 % (w/v), and heat treatment was carried out at 45°C at the same time;
(2) only heat treatment at 45°C was carried out;
(3) Triton X-100 was added to the pretreatment liquid at a concentration of 0.1% (w/v), and heat treatment was carried out at 30°C at the same time; and
(4) only heat treatment at 30°C was carried out.
The treatment time was 3 hours in each case, and the other conditions were the same as in Example 1.
The reaction step was carried out in the same manner as in Example 1. However, glucose was added to have a concentration of 0.25% by weight. Further, the reaction temperature was the same as that in the pretreatment step, and the reaction time was set to 20 hours. Quantification of glucose was carried out in the same manner as in Example 2. The results are shown in Table 4.

**Table 4**

| Reaction time [hrs] | Glucose concentration [g/L] | | | |
|---|---|---|---|---|
| | 1. Addition of surfactant/ heat treatment at 45°C | 2. Only heat treatment at 45°C | 3. Addition of surfactant/ heat treatment at 30°C | 4. Only heat treatment at 30°C |
| 0 | 24.5 | 24.7 | 24.7 | 23.7 |
| 20 | 25.0 | 21.0 | 20.6 | 6.8 |

As shown in Table 4, it was found that exposure of the arming yeast to a surfactant (Triton X-100) generated an effect on the glucose metabolic capability of the arming yeast that is similar to that generated by heat treatment at 45°C. It was also found that the glucose metabolism of the arming yeast can be completely suppressed by carrying out both exposure to a surfactant and heat treatment at 45°C.

### Example 5

### (Induction of glucose accumulation of arming yeast by surfactant and heat treatment)

In the same manner as in Example 4, the arming yeast presenting cellulase at the cell surface layer (MT8-1/EG, CBH, BGL strain) was cultured, washed, pretreated and subjected to a reaction step. However, Triton X-100 at a concentration of 1% (w/v) as a surfactant was added to the pretreatment liquid, and the pretreatment was carried out at a pretreatment temperature of 45°C for a treatment time of 24 hours. In the reaction step, the cellulose swollen by phosphoric acid was added to have a concentration of 2% by weight, and the reaction was carried out at a temperature of 45°C for a reaction time of 139 hours. Quantification of glucose was carried out in the same manner as in Example 4. A sample that did not contain a surfactant was used as a control group. The results are shown in Table 5.

**Table 5**

| Reaction time [hrs] | Glucose concentration | |
|---|---|---|
| | [g/L] | |
| | Without addition of Triton X-100/ Heat treatment at 45°C | Addition of 1% Triton X-100/ Heat treatment at 45°C |
| 0 | 0.1 | 0.14 |
| 139 | 6.0 | 11.7 |

As shown in Table 5, the concentration of accumulated glucose was 6 g/L in the case in which only the heat treatment was carried out; in the case in which both of the heat treatment and the exposure to Triton X-100 were carried out, the concentration almost doubled to 11.7 g/L. Thus, it was found that the glucose accumulation ability of the arming yeast can be drastically enhanced by carrying out both of exposure to a surfactant (Triton X-100) and heat treatment at 45°C in the pretreatment step.

### Example 6

### (Difference in induction of decreased in glucose metabolic capability of arming yeast between types of surfactant)

In the same manner as in Example 5, the arming yeast presenting cellulase at the cell surface layer (MT8-1/EG, CBH, BGL strain) was cultured, washed, pretreated and subjected to a reaction step. However, each of the following surfactants was added at a concentration of 1 % (w/v) to mutually different pretreatment liquids. The surfactants used were: (1) NONIDET P-40 (polyoxyethylene nonylphenyl ether, manufactured by NACALAI TESQUE), (2) TWEEN 85 (polyoxyethylene sorbitan trioleate, manufactured by Tokyo Chemical Industry Co., Ltd.), (3) ARLACEL C (sorbitan sesquioleate, manufactured by Tokyo Chemical Industry Co., Ltd.), (4) TWEEN 20 (polyoxyethylene sorbitan monolaurate, manufactured by Junsei Chemical Co., Ltd.), (5) TWEEN 80 (polyoxyethylene sorbitan monooleate, manufactured by Kishida Chemical Co., Ltd.), (6) TRITON X-100 (4-(1,1,3,3-tetramethylbutyl)phenyl-polyethyleneglycol, manufactured by Wako Pure Chemical Industries, Ltd.), (7) SPAN 85 (sorbitan trioleate, manufactured by Tokyo Chemical Industry Co., Ltd.) and (8) SPAN 80 (sorbitan monooleate, manufactured by Kishida Chemical Co., Ltd.). The pretreatment was carried out at 45°C for 3 hours in all cases. After the pretreatment, the reaction liquid was incubated at 30°C for 30 minutes, and then the reaction step was conducted. In the reaction step, glucose was added to have a concentration of 0.5% by weight, the temperature was set to 30°C, and the reaction time was set to 44 hours. Quantification of glucose was carried out in the same manner as in Example 4. A sample that did not contain a surfactant was used as a control group. The results are shown in Table 6.

**Table 6**

| Reaction time [hrs] | Glucose concentration | | | | | | | | |
|---|---|---|---|---|---|---|---|---|---|
| | [g/L] | | | | | | | | |
| | 1 | 2 | 3 | 4 | 5 | 6 | 7 | 8 | Without surfactant |
| | NONIDET | TWEEN | ARLACEL | TWEEN | TWEEN | TRITON | SPAN | SPAN | |
| | P-40 | 85 | C | 20 | 80 | X-100 | 85 | 80 | |
| 0 | 4.9 | 4.8 | 4.5 | 4.8 | 4.5 | 4.9 | 4.6 | 4.1 | 4.6 |
| 44 | 4.4 | 3.9 | 3.4 | 0.6 | 0.0 | 4.7 | 0.2 | 2.9 | 0.0 |

As shown in Table 6, it was found that various surfactants induce a decrease in the glucose metabolic capability of the arming yeast. In particular, (1) NONIDET P-40, (2) TWEEN 85, (3) ARLACEL C, (6) TRITON X-100 and (8) SPAN 80 were found to have high effects in terms of suppression of glucose uptake.

### Example 7

### (Induction of decrease in glucose metabolic capability of arming yeast by exposure to antibiotic substance)

In the same manner as in Example 5, the arming yeast presenting cellulase at the cell surface layer (MT8-1/EG, CBH, BGL strain) was cultured, washed, pretreated and subjected to the reaction step. However, ZEOCIN (manufactured by Invitrogen; recommended concentration for use: 5 to 300 µg/mL), which is an antibiotic substance effective in eukaryotic organisms, was added to the pretreatment liquid at a concentration of 200 µg/mL instead of a surfactant, and the pretreatment was carried out at a pretreatment temperature of 30°C for treatment time of 24 hours. In general, yeast cannot grow at this concentration. In the reaction step, glucose was added to have a concentration of 0.5% by weight, and the reaction was carried out at 45°C for a reaction time of 6 hours. Quantification of glucose was carried out in the same manner as in Example 4. A sample that was not subjected to the pretreatment step was used as a control group. The results are shown in Table 7.
As shown in Table 7, it was found that addition of ZEOCIN in the pretreatment step cannot induce a decrease in glucose metabolic capability of the arming yeast at all. Thus, it was found that exposure of the yeast to a lethal amount of an antibiotic substance cannot induce a decrease in glucose metabolism

**Table 7**

| Reaction time [hrs] | Glucose concentration | |
|---|---|---|
| | [g/L] | |
| | Without pretreatment | ZEOCIN added |
| 0 | 5.0 | 5.2 |
| 6 | 0.0 | 0.0 |

### Example 8

### (Heat Resistance Test of Arming Yeast)

With regard to the arming yeast catalyst according to the invention, it is important that the shape of the yeast, which is a carrier for presenting the cellulase, be maintained in order to enable the cellulase presented on the surface to function appropriately and in order to use the catalyst repeatedly. Thus, the heat treatment temperature at which the arming yeast can maintain its normal shape was examined as follows.

In the same manner as in Example 3, the arming yeast presenting cellulase at the cell surface layer (MT8-1/EG, CBH, BGL strain) was cultured and washed. However, the culture medium used was a YPD medium. 0.1 mL of a 300 g/L yeast liquid and 0.9 mL of pure water were added into a 1.5 mL Eppendorf tube, and the resulting mixture was stirred and then aliquoted in 0.1 mL volumes to 0.3mL plastic tubes. The aliquots were heat-treated at 30, 45, 50, 60, 70, 80 and 90°C, respectively, and cooled to 4°C. Thereafter, the number of cells that maintained normal morphology was counted using a hemacytometer (manufactured by Neubauer, 1/400 mm²; 1/10 mm depth). The measurement of the cell number was carried out at 160 areas each having an area of 1/400 mm², and the average thereof was calculated. A sample that was not subjected to heat treatment was used as a control group. The numbers of normal cells in the samples treated at the respective temperatures, as indicated assuming the number of cells in the sample that was not subjected to heat treatment as 100, are shown in Table 8.
As shown in Table 8, it was found that the arming yeast gradually becomes unable to maintain its normal morphology at heat treatment temperatures of 60°C or higher, and the proportion of cells having normal morphology is 22% or lower at temperatures of 70°C or higher.

**Table 8**

| Proportion of cells with normal morphology [%] | Heat treatment temperature | | | | | | | |
|---|---|---|---|---|---|---|---|---|
| | [°C] | | | | | | | |
| | No heat treatment | 30°C | 45°C | 50°C | 60°C | 70°C | 80°C | 90°C |
| | 100 | 86 | 88 | 89 | 62 | 22 | 15 | 10 |

### Example 9

### (Effect with respect to recovery of glucose metabolic capability of arming yeast of which glucose metabolic capability was decreased by heat treatment)

The arming yeast catalyst according to the invention aims at accumulating glucose in a reaction liquid. Therefore, recovery of the glucose metabolic capability, which is once decreased by heat in the pretreatment step, during the reaction step is inconvenient from the viewpoint of glucose productivity. Therefore, the temperature range in which the glucose metabolic capability of the pretreated arming yeast is not recovered was examined as follows.

In the same manner as in Example 3, the arming yeast presenting cellulase at the cell surface layer (MT8-1/EG, CBH, BGL strain) was cultured, washed, pretreated and subjected to the reaction step. However, the culture medium used was a YPD medium. The pretreatment was carried out at a pretreatment temperature of 45°C for treatment time of 24 hours. Thereafter, incubation was carried out at varied temperatures of 25°C, 35°C, 45°C and 55°C for varied periods of 0 hour, 22 hours, 47 hours and 76 hours, and then each sample was subjected to a reaction step. In the reaction step, the cellulose swollen by phosphoric acid was added to have a concentration of 1% by weight, and the reaction was carried out at varied temperatures of 25°C, 35°C, 45°C and 55°C for a reaction time of 48 hours. Quantification of glucose was carried out in the same manner as in Example 4. The concentrations of accumulated glucose are shown in Table 9.

As shown in Table 9, it was found that the glucose metabolic capability of the arming yeast is recovered by decreasing the temperature to 25°C or 35°C after the pretreatment with heat, so that the yeast starts to consume glucose. In contrast, it was found that the effect with respect to the decrease in glucose metabolic capability is maintained by maintaining the temperature at 45°C or higher after the pretreatment.

**Table 9**

| Incubation time [hrs] | Incubation temperature after pretreatment [°C] | | | |
|---|---|---|---|---|
| | 25 | 35 | 45 | 55 |
| 0 | 0.2 | 0.2 | 3.3 | 4.7 |
| 22 | 0.1 | 0.6 | 3.7 | 3.6 |
| 47 | 0.0 | 0.1 | 3.8 | 2.9 |
| 76 | 0.0 | 0.1 | 3.0 | 2.4 |

### Example 10

### (Recycling Test of Arming Yeast)

The following test was performed to examine whether the arming yeast catalyst according to the invention can be recovered and recycled after the arming yeast catalyst was used for saccharification of cellulose.
In the same manner as in Example 3, the arming yeast presenting cellulase at the cell surface layer (MT8-1/EG, CBH, BGL strain) was cultured, washed, pretreated and subjected to the reaction step. However, the culture medium used was a YPD medium. The pretreatment was carried out at 45°C for a reaction time of 24 hours. In the reaction step, the cellulose swollen by phosphoric acid was added to have a concentration of 2% by weight, and the reaction was carried out at a temperature of 45°C for a reaction time of 24 hours. After completion of the reaction step in the first round, the arming yeast was collected by centrifugal separation treatment, and the collected yeast was suspended in a reaction liquid containing the phosphoric-acid-swollen cellulose containing the same amount of glucose as the amount of glucose produced by the initial reaction step, and the reaction step was carried out again. In the same manner, the collection of the arming yeast and the reaction step were repeated, such that recycling was carried out five times in total. Quantification of glucose was carried out in the same manner as in Example 4.

The remaining activity in each recycle reaction step was calculated as follows. The glucose concentrations at 0 hour and 24 hours in each reaction step were measured, and the amount of glucose generated in each recycle reaction step was calculated. The amount of glucose generated was divided by each reaction time, thereby calculating the glucose production rate per hour, which was regarded as the catalytic activity in each step. The catalytic activity in each recycle step was represented as a relative value assuming that the catalytic activity in the first round as 100, and the obtained relative value was regarded as the remaining activity. Further, as a control, a similar test was carried out using a purified cellulase (ACCELLERASE; manufactured by Genencor) at a concentration of 0.1 % (w/v) instead of the arming yeast. The results are shown in Table 10.

**Table 10**

| Number of Recycling cycles [times] | Remaining activity [%] | |
|---|---|---|
| | Arming yeast | ACCELLERASE |
| 0 | 100.0 | 100.0 |
| 1 | 58.4 | 15.8 |
| 2 | 68.3 | 8.8 |
| 3 | 70.3 | 1.3 |
| 4 | 48.6 | 1.5 |
| 5 | 48.3 | 0.0 |

As shown in Table 10, the arming yeast maintained at least 48% of its catalytic activity even after five times of recycling. In contrast, the catalytic activity of ACCELLERASE drastically decreased as the number of recycling cycles increased, and hardly any activity remained in the third and subsequent cycles. From these, it was confirmed that a purified enzyme cannot be recycled whereas the arming yeast catalyst can be recovered and used for the saccharification reaction from cellulose to glucose many times.

### Example 11

### (Recycling Test of Arming Yeast at 60°C)

Whether it is possible to maintain the morphology of the yeast in the case of recycling the arming yeast catalyst according to the invention at 60°C was examined in the following manner.
In the same manner as in Example 8, the arming yeast presenting cellulase at the cell surface layer (MT8-1/EG, CBH, BGL strain) was cultured and washed. 0.1 mL of a 300 g/L yeast liquid and 0.9 mL of pure water were added into a 2.0 mL Eppendorf tube, and incubated at 60°C while stirring at a rotation speed of 1000 rpm/min. (BIOSHAKER, M-BR-022UP; manufactured by TAITEK). A 0.01 mL aliquot was collected every 24 hours and subjected to centrifugation treatment, and the supernatant was removed. Thereafter, the resultant was resuspended in 1 mL of pure water, and cooled to 4°C. The number of cells that maintained normal morphology was measured using a hemacytometer (manufactured by Neubauer, 1/400 mm²; 1/10 mm depth). The measurement of the cell number was carried out at 160 areas each having an area of 1/400 mm², and the average value thereof was calculated. A sample that was not subjected to heat treatment was prepared as a control group. The results with respect to the number of normal cells in the recycled sample, assuming that the number of cells in the sample that was not subjected to heat treatment is 100, are shown in Table 11.
As shown in Table 11, in the case in which the arming yeast was recycled at 60°C, the proportion of yeast cells maintaining normal morphology after the fifth recycling cycle was 26%.

**Table 11**

| Number of recycling cycles [times] | Proportion of cells with normal morphology [%] |
|---|---|
| 0 | 100 |
| 1 | 72 |
| 2 | 44 |
| 3 | 40 |
| 4 | 30 |
| 5 | 26 |

### Example 12

### (Recycling Test of Arming Yeast at 50°C)

Whether it is possible to maintain the morphology of the yeast in the case of recycling the arming yeast catalyst according to the invention at 50°C was examined in the following manner.
In the same manner as in Example 11, the arming yeast presenting cellulase at the cell surface layer (MT8-1/EG, CBH, BGL strain) was cultured and washed. Thereafter, a recycling experiment on the arming yeast was carried out in the same manner as in Example 11. However, the incubation temperature was 50°C, and the number of recycling cycles was 7. As a control group, a sample that was not subjected to heat treatment was prepared. The results with respect to the number of normal cells in the sample after recycling, assuming that the number of cells in the sample that was not subjected to heat treatment is 100, are shown in Table 12.
As shown in Table 12, in the case in which the arming yeast was recycled at 50°C, 56% of the yeast cells maintained its normal morphology even after completion of the seventh recycling cycle.

**Table 12**

| Number of recycling cycles [times] | Proportion of cells with normal morphology [%] |
|---|---|
| 0 | 100 |
| 1 | 90 |
| 2 | 69 |
| 3 | 67 |
| 4 | 77 |
| 5 | 67 |
| 6 | 62 |
| 7 | 56 |

### Example 13

### (Production of Glucose by Arming Yeast from Raw Pulp Material)

Glucose was produced from kraft pulp in the following manner, using the arming yeast according to the invention and a yeast produced by allowing the arming yeast according to the invention to further express a cellobiohydrolase derived from *Cellulomonas fimi* (Cex).

### <1> Construction of arming yeast obtained by allowing the MT8-1/EG, CBH, BGL strain to express cellobiohydrolase derived from Cellulomonas fimi (Cex)

The base sequence of cellobiohydrolase derived from *Cellulomonas fimi* (Cex) has already been reported (GenBank accession number M15199). Two types of primers, CGCGGCAGAAACGAGCAAAGAAAAGTAAG (SEQ ID NO:1) and AGATCTCCATGGCTCGAGCGCCAAAAGCTC (SEQ ID NO:2), for amplifying the vector region of pEG23u31 H6 (described in Fujita et.al., Applied and Environmental Microbiology, Vol.68 (10), pp.5136-5141 (2002)), which is a plasmid for expressing an anchor protein, were constructed. Further, two types of primers, TACTTTTCTTTGCTCGTTTCTGCCGCGGGAGCGACCACGCTCAAGGAGGC (SEQ ID NO:3) and GCTTTTGGCGCTCGAGCCATGGAGATCTAGGCCGACCGTGCAGGGCGTG (SEQ ID NO:4), which have, at 5'-ends thereof, sequences complementary to the above primers and have, at 3'-ends thereof, sequences homologous to the mature protein portion of Cex were constructed.

PCR was carried out under normal conditions using pEG23u31 H6 as a template and the primers of SEQ ID NO:1 and SEQ ID NO:2, thereby obtaining a DNA fragment of about 10 kbp (hereinafter referred to as "pEG23u31H6 fragment"). Further, PCR was carried out under normal conditions using a genomic gene of the *Cellulomonas fimi* ATCC484 strain as a template and the primers of SEQ ID NO:3 and SEQ ID NO:4, thereby amplifying a DNA fragment of about 1.3 kbp (hereinafter referred to as "Cex fragment"). Using a mixed solution of the pEG23u31 H6 fragment and the Cex fragment obtained, the MT8-1/EG, CBH, BGL strain prepared using a Fast Yeast Transformation Kit (manufactured by Takara Bio Inc.) was transformed, and the resulting transformants were applied to a SD plate medium that does not contain uracil, and cultured at 30°C for 5 days. The obtained transformants were named MT8-1/EG,CBH,BGL/pCex strain.
The *Cellulomonas fimi* ATCC484 strain can be obtained from American Type Culture Collection, which is a cell/microorganism/gene bank.

### <2> Test of Glucose Production from Kraft Pulp by MT8-1/EG,CBH,BGL Strain and MT8-1/EG,CBH,BGL/pCex Strain

First, kraft pulp was prepared as follows.
A sheet-shaped kraft pulp with a.content of 50% (dry w/w) (derived from hardwood; bleached; manufactured by NIPPON DAISHOWA PAPERBOARD Co,. Ltd.) was torn into small pieces, and added into a blender so as to be suspended at a concentration of 7% (dry w/w) in sterilized water. This was subjected to sterilization treatment using an autoclave, and a portion thereof was collected, and dry weight thereof was measured. The pulp after autoclaving was further diluted with sterilized water to a concentration of 3% (dry w/w) and suspended, and the pH was adjusted to 4.8 with a 100 mM citrate buffer (pH 4.8) solution, thereby providing a pulp stock.

In the same manner as in Example 5, the arming yeast was cultured, washed, pretreated and subjected to the reaction step. However, two types of arming yeast, MT8-1/EG,CBH,BGL strain and MT8-1/EG,CBH,BGL/pCex strain, were prepared. Further, as a control, a host yeast (MT8-1) that does not present cellulase at the surface layer was prepared. To the pretreatment liquid, Triton X-100 was added as a surfactant at a concentration of 0.1% (w/v), and the pretreatment was carried out at a temperature of 45°C for treatment time of 24 hours. In the reaction step, the kraft pulp was added to have a concentration of 0.5% by weight, and the reaction was carried out at a temperature of 45°C for a reaction time of 216 hours. Quantification of glucose was carried out in the same manner as in Example 4. The results are shown in Table 13.

As shown in Table 13, it was demonstrated that glucose can be produced from kraft pulp by the arming yeast catalyst according to the invention. Further, it was found that the glucose productivity is increased by presenting a different type of cellulase (for example, cellobiohydrolase derived from *Cellulomonas fimi* (Cex)) at the surface layer of the arming yeast according to the invention.

**Table 13**

| Reaction time [hrs] | Glucose concentration | | |
|---|---|---|---|
| | [g/L] | | |
| | MT8-1/EG,CBH,BGL/pCex strain | MT8-1/EG,CBH,BGL strain | MT8-1 strain |
| 0 | 0.01 | 0.00 | 0.00 |
| 72 | 0.18 | 0.10 | 0.00 |
| 96 | 0.29 | 0.14 | 0.00 |
| 120 | 0.32 | 0.19 | 0.00 |
| 144 | 0.41 | 0.22 | 0.00 |
| 168 | 0.50 | 0.41 | 0.00 |
| 216 | 0.69 | 0.44 | 0.00 |

As described above, according to the invention, the glucose metabolic activity can be easily decreased by subjecting the arming yeast to pretreatment at a predetermined temperature condition or pretreatment by exposure to a surfactant. Use of the pretreated arming yeast enables glucose to be accumulated in a reaction liquid at high concentrations. Further, the arming yeast according to the invention can be easily recovered from the reaction liquid in which glucose has been accumulated, and recycled for further use.
Therefore, glucose can be efficiently obtained from cellulose at high yields by repeatedly using such an arming yeast of which the glucose metabolic activity is decreased.

The disclosure of Japanese Patent Application No. 2008-105050 is incorporated by reference herein in its entirety.
All publications, patent applications, and technical standards mentioned in this specification are herein incorporated by reference to the same extent as if each individual publication, patent application, or technical standard was specifically and individually indicated to be incorporated by reference.

## Claims

1. A yeast having a cellulose degradation ability that degrades cellulose outside a cell thereof and a glucose accumulation ability that accumulates, in a reaction liquid, glucose produced from cellulose as a result of the cellulose degradation ability when the yeast contacts with cellulose in the reaction liquid.

2. The yeast according to claim 1, wherein the glucose accumulation ability is based on decreased activity of glucose metabolism.

3. The yeast according to claim 1 or 2, wherein the glucose accumulation ability is induced by heat treatment at a temperature of from more than an optimum growth temperature of the yeast to less than a denaturation temperature of a cellulolytic enzyme that imparts the cellulose degradation ability.

4. The yeast according to any one of claims 1 to 3, wherein the glucose accumulation ability is induced by heat treatment at a temperature of from 40°C to 70°C.

5. The yeast according to any one of claims 1 to 4, wherein the glucose accumulation ability is induced by a treatment using a surfactant.

6. The yeast according to any one of claims 1 to 5, wherein the glucose accumulation ability is induced by a treatment of contacting the yeast with a surfactant solution at a concentration of from 0.015% (w/v) to 10% (w/v).

7. The yeast according to any one of claims 1 to 6, wherein the yeast is a *Saccharomyces* yeast.

8. The yeast according to any one of claims 1 to 7, wherein the yeast is yeast comprising a cellulolytic enzyme in a surface layer thereof.

9. A glucose production method of producing glucose from cellulose, the method comprising:
a step of contacting the yeast according to any one of claims 1 to 8 with cellulose in a reaction liquid; and
a step of separating and collecting, from the reaction liquid, glucose produced and accumulated in the reaction liquid as a result of the contact between the yeast and the cellulose.

10. The method according to claim 9, the method further comprising contacting the yeast in the reaction liquid after the step of separating and collecting with newly-supplied cellulose, wherein the yeast in the reaction liquid after the separating and collecting is used repeatedly at least once.

11. The method according to claim 9 or 10, further comprising collecting the yeast in the reaction liquid after the step of separating and collecting.

12. The method according to any one of claims 9 to 11, further comprising carrying out a pretreatment for inducing the glucose accumulation ability, prior to the step of contacting with cellulose.

13. The method according to claim 12, wherein the pretreatment is a heat treatment at a temperature of from 40°C to 70°C or a treatment with a surfactant.
